# EUROPÄISCHE PATENTANMELDUNG

(11) **EP 2 428 559 A1**
(43) Veröffentlichungstag der Anmeldung: **14.03.2012**
(21) Anmeldenummer: 11007360.8
(22) Anmeldetag: 09.09.2011
(51) Int. Cl.: C12M 1/107

(54) **Biogasanlage, Verfahren zu deren Betreiben sowie Reaktoreinheit hierfür**

(30) Priorität: 10.09.2010 DE 102010044988
(71) Anmelder: Pöttinger Entsorgungstechnik GmbH & Co. KG, 4710 Grieskirchen (AT)
(72) Erfinder: Erler, Johann, 4742 Pram (AT); Zehetner, Johann, 4710 Pollham (AT); Eisner, Holger, 4812 Pinsdorf (AT)
(74) Vertreter: Thoma, Michael

(57) **Zusammenfassung**

Die vorliegende Erfindung betrifft ein Verfahren sowie eine Biogasanlage zur Erzeugung von Biogas aus organischen Abfällen, mit zumindest einer Reaktoreinheit, die einen Reaktorbehälter zur Aufnahme des zu behandelnden Abfalls sowie Funktionsaggregate zur Reaktionssteuerung aufweist, ferner mit einem Gasspeicher zur Zwischenspeicherung des erzeugten Gases sowie einer Steuereinheit zur Steuerung und/oder Überwachung der Funktionsaggregate der Reaktoreinheit. Erfindungsgemäß ist zumindest eine Reaktoreinheit als mobile Fahrzeugcontainereinheit ausgebildet, die mit lösbaren Kupplungsmitteln umfassend eine Gasanschlusskupplung zum Ankuppeln an den Gasspeicher und eine Signalleitungs- und/oder Energieversorgungskupplung zum Ankuppeln an die Steuereinheit versehen ist. Durch die Ausbildung der Reaktoreinheiten als mobile Fahrzeugcontainereinheiten können die Reaktoreinheiten rasch zwischen verschiedenen Biogasanlagen bzw. Standorten hin und her transportiert werden, je nachdem an welchem Standort bzw. an welcher Biogasanlage Kapazitätsspitzen auftreten und zusätzliche Prozessorkapazität benötigt wird.

## Beschreibung

Die vorliegende Erfindung betrifft ein Verfahren sowie eine Biogasanlage zur Erzeugung von Biogas aus organischen Abfällen, mit zumindest einer Reaktoreinheit, die einen Reaktorbehälter zur Aufnahme des zu behandelnden Abfalls sowie Funktionsaggregate wie eine Perkolationseinrichtung zur Zugabe von Perkolat, eine Belüftungseinrichtung zur Steuerung der Luftmenge im Reaktorbehälter, eine Heizeinrichtung zur Beheizung des im Reaktor befindlichen Abfalls und/oder eine Gassensorik zur Erfassung der Gaszusammensetzung in dem Reaktorbehälter aufweist, ferner mit einem Gasspeicher zur Zwischenspeicherung des erzeugten Gases sowie einer Steuereinheit zur Steuerung und/oder Überwachung der Funktionsaggregate der Reaktoreinheit. Die Erfindung betrifft dabei insbesondere die Reaktoreinheit für eine solche Biogasanlage.

Organische Abfälle, die über eine für die Kompostierung bzw. Vergärung genügend großen Anteil biologisch abbaubarer Stoffe verfügen, wie beispielsweise Bioabfällen, Garten- und Parkabfälle einschließlich Grünschnitt, Klär- und Fäkalschlämme, Marktabfälle, Abfällen aus der Lebensmittelverwertung und dergleichen können in an sich bekannter Weise zur Erzeugung von brennbaren Biogasen, insbesondere Methangas genutzt werden. Neben der Produktion von Biogas fällt bei der Behandlung der Abfälle durch aerobe und anaerobe Verfahrensschritte auch Kompost an, der ebenfalls weiterverwertet werden kann. Vorteilhafterweise kann hierbei zunächst in einer aeroben Anlaufphase das Abfallgut auf eine Zieltemperatur gebracht werden und sodann in einer anaeroben zweiten Prozessphase durch Zusetzen von Anaerobbakterien die Methanbildung initiiert werden, wobei üblicherweise Perkolat beispielsweise in Form von Sickerwasser in den Reaktor eingesprüht wird. Die Prozessbedingungen in dem Reaktorbehälter werden hierbei über Funktionsaggregate gesteuert, die insbesondere eine Perkolationseinrichtung zur Zugabe des besagten Perkolats, eine Belüftungseinrichtung zur Steuerung der Luftmenge in dem Reaktorbehälter, eine Heizeinrichtung zur Beheizung des Reaktorbehälters bzw. des darin befindlichen Abfalls und Erfassungseinrichtungen wie Temperatursensoren, Gassensoren zur Messung der Gaszusammensetzung und -konzentration und andere Sensoren zur Erfassung relevanter Betriebsparameter umfassen können.

Derartige Biogasanlagen werden üblicherweise in Form von Großanlagen betrieben, um die für die Erzeugung signifikanter Biogasmengen notwendigen Abfallmengen verarbeiten zu können. Die Reaktoreinheiten bzw. deren Reaktorbehälter sind in der Regel betonierte Boxen, die über geeignete Förderanlagen befüllt und entleert werden können. Hierbei ist es bekannt, mehrere Reaktoreinheiten in einer Biogasanlage zusammenzufassen und miteinander so zu verschalten, dass in einem Reaktor anfallende Prozessprodukte als Zugabestoffe in einem anderen Reaktor verwendet werden können, vgl. DE 44 09 487 C2.

Das Betreiben solcher Biogasanlagen ist allerdings hinsichtlich der Ausnutzung der Kapazität der Anlage und der Produktionseffizienz bislang nicht ausreichend optimiert. Insbesondere macht es Probleme, die Schwankungen der zu verarbeitenden Abfallmenge aufzufangen bzw. die Anlagenkapazität an diese Schwankungen anzupassen. Üblicherweise fallen in den Sommermonaten deutlich höhere Bioabfallmengen an als in den restlichen Jahreszeiten, wobei ggf. jedoch auch andere Jahreszeiten Anfallspitzen zeigen können, beispielsweise die Wintersaison in Wintertourismusgebieten. Wird die Bioabgasanlage für die maximalen, anfallenden Abfallmengen ausgelegt, arbeitet die Anlage über größere Strecken ineffizient, wobei durch eine Überdimensionierung der Reaktorbehälter sogar Probleme beim Ablauf des Prozesses selbst entstehen können, beispielsweise hinsichtlich der Prozesstemperaturen, der Gaszusammensetzung oder der pro Abfallmenge erzeugten Gasmenge. Umgekehrt bedeutet eine Auslegung der Anlage für die durchschnittlich anfallende Abfallmenge oder die mit Ausnahme der Spitzenzeiten anfallenden Normalmenge, dass die in Spitzenzeiten anfallende Abfallmenge nicht vollständig bearbeitet werden kann, zumindest nicht ohne signifikante zeitliche Verzögerung und entsprechende Zwischenlagerung der Abfälle, die zu zusätzlichen Problemen wie Geruchsbelästigung oder Veränderung des Abfallgutes führt.

Der vorliegenden Erfindung liegt hiervon ausgehend die Aufgabe zugrunde, eine verbesserte Biogasanlage sowie eine verbesserte Reaktoreinheit hierfür zu schaffen, die Nachteile des Standes der Technik vermeidet und Letzteren in vorteilhafter Weise weiterbildet. Insbesondere soll auch bei variierenden Abfallmengen eine gleichmäßig gute Auslastung der Anlage erreicht und eine effiziente Biogasproduktion ermöglicht werden.

Erfindungsgemäß wird diese Aufgabe durch eine Biogasanlage nach Anspruch 1, ein Verfahren nach Anspruch 10 sowie eine Reaktoreinheit nach Anspruch 11 gelöst. Bevorzugte Ausgestaltungen der Erfindung sind Gegenstand der abhängigen Ansprüche.

Es wird also vorgeschlagen, der Biogasanlage einen modularen Aufbau zu geben, um je nach Kapazitätsbedarf eine unterschiedliche Anzahl von Reaktoreinheiten anschließen zu können. Erfindungsgemäß ist zumindest eine Reaktoreinheit als mobile Fahrzeugcontainereinheit ausgebildet, die mit lösbaren Kupplungsmitteln umfassend eine Gasanschlusskupplung zum Ankuppeln an den Gasspeicher und eine Signalleitungs- und/oder Energieversorgungskupplung zum Ankuppeln an die Steuereinheit versehen ist. Die Biogasanlage kann vorteilhafterweise auf die durchschnittliche Hauptlast ausgelegt werden, wobei zur Bewältigung von Kapazitätsspitzen zusätzliche Reaktoreinheiten in Form mobiler Fahrzeugcontainereinheiten zusätzlich angeschlossen werden können. Durch die Ausbildung der Reaktoreinheiten als mobile Fahrzeugcontainereinheiten können die Reaktoreinheiten rasch zwischen verschiedenen Biogasanlagen bzw. Standorten hin und her transportiert werden, je nachdem an welchem Standort bzw. an welcher Biogasanlage Kapazitätsspitzen auftreten und zusätzliche Prozessorkapazität benötigt wird. Hierdurch können mit einer an sich begrenzten Anzahl an Reaktoreinheiten an verschiedenen Standorten Kapazitätsspitzen, die üblicherweise nicht an allen Standorten gleichzeitig auftreten, abgefangen werden, wobei die jeweiligen Anlagenstandorte mit einer Grundausstattung nur für die durchschnittliche Hauptlast ausgerüstet sein müssen.

In Weiterbildung der Erfindung kann auch der Gasspeicher und/oder die Steuereinheit, an die die genannten Reaktoreinheiten anschließbar sind, in Form einer mobilen Fahrzeugcontainereinheit ausgebildet sein, die mit lösbaren Kupplungsmitteln umfassend mehrere Gasanschlusskupplungen und mehrere Signalleitungs-und/oder Energieversorgungskupplungen zum Ankuppeln mehrerer Reaktoreinheiten versehen ist. Durch die Ausbildung auch der Steuereinheit und des Gasspeichers in Form einer mobilen Fahrzeugcontainereinheit wird eine noch größere Flexibilität erzielt, die es erlaubt, je nach benötigter Kapazität einen zusätzlichen Gasspeicher und/oder eine zusätzliche Steuereinheit rasch an den benötigten Standort zu verbringen. Vorteilhafterweise sind der Gasspeicher und/oder die Steuereinheit jeweils mit einer ausreichenden Vielzahl von lösbaren Anschlusskupplungen versehen, um eine Vielzahl von Reaktoreinheiten an den Gasspeicher und/oder die jeweilige Steuereinheit anschließen zu können. Vorteilhafterweise sind an dem Gasspeicher und/oder der Steuereinheit jeweils mindestens drei, vorzugsweise mehr als fünf und insbesondere zehn oder mehr Kupplungsmittel zum Ankuppeln einer entsprechenden Anzahl von Reaktoreinheiten vorgesehen.

Vorteilhafterweise ist die Steuereinheit in der Lage, die Bearbeitungsprozesse in mehreren Reaktoreinheiten jeweils individuell zu steuern, um die Bearbeitungsprozesse in verschiedenen Reaktoreinheiten zeitlich unterschiedlich ablaufen lassen zu können, je nachdem, wann ein Reaktionsprozess in einem jeweiligen Reaktorbehälter in Gang gesetzt wurde. Die Steuereinheit kann hierzu entsprechende Steuerungsmodule besitzen, von denen jeweils eines für die Steuerung einer Reaktoreinheit vorgesehen ist. Vorteilhafterweise sind die Steuerungsmodule hierbei derart ausgebildet, dass die Bearbeitungsprozesse in mehreren Reaktoren zeitlich überlappend oder auch zeitlich nicht überlappend unabhängig voneinander gesteuert werden können, um je nach Kapazitätsbedarf und anfallender Abfallmenge einen zusätzlichen Reaktor in Betrieb nehmen zu können, ungeachtet dessen, in welchem Stadium sich die Reaktionsprozesse in den anderen Reaktoren befinden.

Vorteilhafterweise erkennt die Steuereinheit selbständig den Anschluss einer weiteren Reaktoreinheit. Durch den Anschluss der Signalleitungs- bzw. Energieversorgungskupplung eines Reaktors an die Steuereinheit initiiert die Steuereinheit automatisch eine entsprechende Identifikationsroutine, um die Funktionsaggregate des Reaktors und/oder den Typ des Reaktors, um die benötigten Steuerungssignale entsprechend anzupassen, und initiiert dann ggf. automatisch oder halbautomatisch die entsprechende Steuerungsroutine. Hierdurch kann die Anbindung der Reaktoreinheiten nach dem Plug-and-play-Prinzip erfolgen. Die Steuereinheit und/oder die Reaktoreinheiten umfassen hierzu entsprechende Erkennungs- und Initiierungsbausteine.

Vorteilhafterweise bildet jede Reaktoreinheit eine selbständige Funktionseinheit, die mit allen für den Reaktionsprozess notwendigen Funktionsaggregaten wie Perkolationseinrichtung, Belüftungseinrichtung, Heizeinrichtung und Sensorik versehen ist, so dass eine jeweilige Prozessoreinheit unabhängig von anderen Prozessoreinheiten arbeiten kann und lediglich die Steuerungssignale von der zentralen Steuereinheit benötigt und das produzierte Gas an einen vorteilhafterweise gemeinsamen Gasspeicher abgibt, an den alle oder eine Untergruppe der Prozessoreinheiten anschließbar sind.

Um eine einfache Handhabung und eine rasche Transportierbarkeit der vorgenannten Transportcontainereinheiten, die die Reaktoreinheiten bilden und/oder die Steuereinheit und/oder Gasspeicher bilden, zu erreichen, können die genannten Transportcontainereinheiten in Form eines Hakencontainers, insbesondere Abroll-Hakencontainer ausgebildet sein und/oder kompatibel zu einem Abrollcontainertransportfahrzeug sein. Insbesondere können die genannten Containereinheiten mit einer Hub- und/oder Zugmittelaufnahme zum Ankuppeln eines Hub- und/oder Zugmittels eines Containerfahrzeugs versehen sein und im Bereich des Bodens der Containereinheit Rollen und/oder Räder besitzen, so dass die Containereinheiten in an sich bekannter Weise einfach von Hakencontainerfahrzeugen aufgeladen, abgeladen und transportiert werden können.

Die Funktionsaggregate einer jeweiligen Transportcontainereinheit können grundsätzlich an verschiedenen Positionen und Abschnitten des jeweiligen Containers angebracht bzw. angeordnet sein. Um die Funktionsaggregate vor Beschädigungen zu schützen, ohne ihre Zugänglichkeit zu beeinträchtigen, und bei vorgegebenen, beispielsweise die Fahrzeugbreite berücksichtigenden Containermaßen das nutzbare Containervolumen möglichst wenig zu beeinträchtigen, ist in vorteilhafter Weiterbildung der Erfindung vorgesehen, dass zumindest ein Teil der Funktionsaggregate und zumindest ein Teil der diesen zugeordneten Anschlusskupplungen an einer Containerstirnseite angeordnet ist, die eine der beiden kleinen Seiten des insgesamt quaderförmigen Containers bildet. Insbesondere können die jeweiligen Funktionsaggregate und die diesen zugeordneten Anschlusskupplungen auf der Containerseite angeordnet sein, an der die vorgenannte Hub- und/oder Zugmittelaufnahme des Hakencontainers vorgesehen ist und mit der voran der Container auf das jeweilige Transportfahrzeug gezogen bzw. gehoben wird.

Bei der die Reaktoreinheit bildenden Transportcontainereinheit kann insbesondere ein Perkolationsmitteltank und/oder zumindest ein Gasanschluss an der genannten Containerstirnseite angeordnet sein, wobei vorteilhafterweise sämtliche Gasanschlüsse und sämtliche Signalleitungs- bzw. Energieversorgungsanschlüsse an derselben Containerstirnseite angeordnet sind. Gegebenenfalls kann auf der genannten Containerseite auch noch ein Ventilator der Belüftungseinrichtung angeordnet sein. Durch die Zusammenfassung der Funktionsaggregate an einer Containerseite wird der vor Beschädigungen zu schützende Bereich der Containereinheit verkleinert; gleichzeitig wird die Bedienung und Wartung der Containereinheit für den Betrieb als Biogasanlage beispielsweise beim Anschließen an die Anlage und/oder der Wartung vereinfacht.

Die Heizeinrichtung zur Beheizung des zu behandelnden Abfallsgutes kann vorteilhafterweise im Bereich des Bodens der Containereinheit vorgesehen sein. Alternativ oder zusätzlich kann im Bereich des Bodens eine Belüftungsleitung der Belüftungseinrichtung vorgesehen sein, die vorteilhafterweise gleichzeitig als Abflussleitung für das bei der Abfallbehandlung anfallende Prozessfluid dienen kann.

Um einerseits der Transportcontainereinheit die notwendige Stabilität zu geben und andererseits den Prozessbetrieb in energiegünstiger Weise zu ermöglichen, kann in vorteilhafter Weiterbildung der Erfindung die Fahrzeugcontainereinheit zumindest abschnittsweise, vorzugsweise an zumindest drei, weiter vorteilhafterweise an zumindest vier Seiten eine mehrschichtige Wandung besitzen, die jeweils zumindest eine Wärmeisolationsschicht und eine Stabilität gebende Stützschicht aufweist. Insbesondere können zumindest die Längsseiten und die Decke, vorzugsweise auch der Boden und ggf. auch die Stirnseiten mit einem solchen mehrschichtigen Wandungsaufbau versehen sein. Vorteilhafterweise kann hier ein sandwichartiger Wandungsaufbau vorgesehen sein, der vorteilhafterweise einen Innenwandung aus Metallblech, eine Außenwandung aus einem schlag- und/oder druckfesten Werkstoff wie beispielsweise einem Metallblech sowie eine zwischen den genannten Innen- und Außenwandungen angeordnete Isolationsschicht aufweist. Durch die Verwendung der genannten Isolationsschicht können die notwendigen Prozesstemperaturen ohne umfangreiche Wärmezufuhr erreicht bzw. gehalten werden, während andererseits die Innen- und Außenwandungen aus druck- und schlagfesten Paneelen dem Container eine ausreichende Steifigkeit bzw. Festigkeit geben, um den Belastungen durch die Abfallbefüllung und den Transportbetrieb zu widerstehen.

Bei der Transportcontainereinheit, die die Steuereinheit und den Gasspeicher bildet, sind die genannte Steuereinheit und der genannte Gasspeicher vorteilhafterweise voneinander getrennt untergebracht und in verschiedenen Abschnitten der Fahrzeugcontainereinheit angeordnet. Insbesondere können der Gasspeicher und die Steuereinheit voneinander getrennt in Form separater Funktionsbausteine ausgebildet sein und verschiedene Abschnitte der Fahrzeugcontainereinheit bilden. Der Korpus der Fahrzeugcontainereinheit kann hierzu in verschiedene Kammern unterteilt sein, in denen jeweils die Steuereinheit oder der Gasspeicher untergebracht sind.

Der genannte Gasspeicher kann vorteilhafterweise eine Kammer mit veränderlichem Volumen besitzen, so dass die Gaseinleitung ohne Druckaufbau und ohne komplizierte Ventiltechnik erfolgen kann. Vorteilhafterweise kann der Gasspeicher in einer Containerkammer untergebracht sein, die zumindest abschnittsweise durch eine verformbare Membran verschlossen ist. Insbesondere kann die Decke der Containereinheit im Bereich des Gasspeichers durch eine verformbare Membran beispielsweise in Form einer gasdichten Textilmembran ausgebildet sein.

Die Erfindung wird nachfolgend anhand eines Ausführungsbeispiels und zugehöriger Zeichnungen näher erläutert. In den Zeichnungen zeigen:
- Fig. 1:: eine schematische, perspektivische Gesamtansicht einer Biogasanlage bestehend aus mehreren mobilen Transportcontainereinheiten, die eine als Gasspeicher und Technikeinheit ausgebildete Containereinheit sowie mehrere daran anschließbare Containereinheiten, die jeweils eine Reaktoreinheit bilden, umfassen,
- Fig. 2:: eine schematische, perspektivische Einzelansicht einer eine Reaktoreinheit bildenden Transportcontainereinheit, die deren Ausbildung als Abroll-Hakencontainer zeigt und die Anordnung einiger Funktionsaggregate an der die Hakenaufnahme umfassenden Containerstirnseite verdeutlicht,

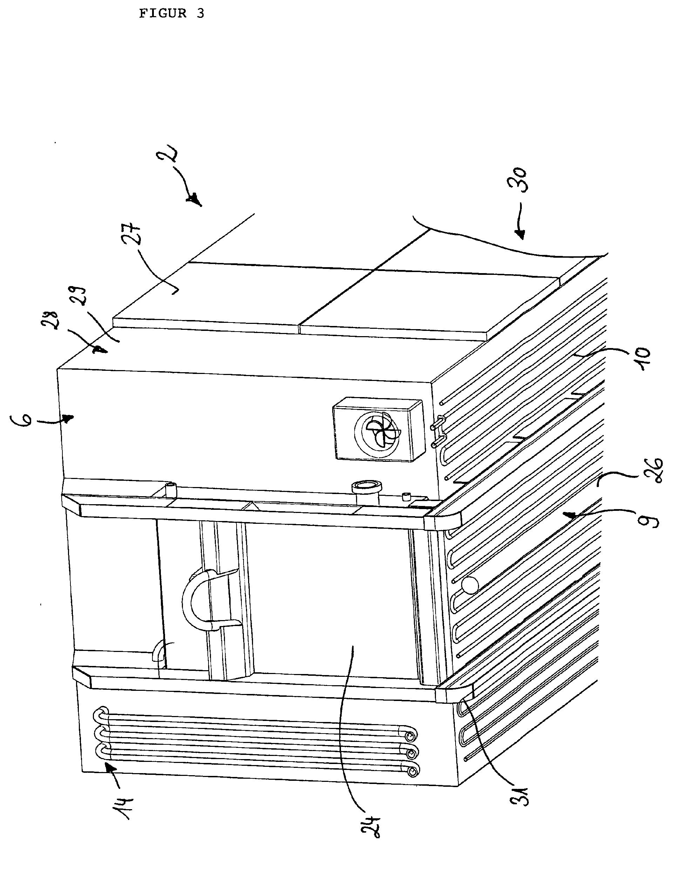

- Fig. 3:: eine ausschnittsweise, perspektivische Ansicht der Reaktoreinheit aus Fig. 2, die die Anordnung einer Heizvorrichtung und einer Belüftungsleitung am Boden der Containereinheit zeigt,
- Fig. 4:: eine schematische Schnittansicht der Reaktoreinheit aus Fig. 2 und 3, und
- Fig. 5:: eine schematische, perspektivische Einzelansicht der als Transportcontainereinheit ausgebildeten Steuereinheit mit angeschlossenem Gasspeicher.

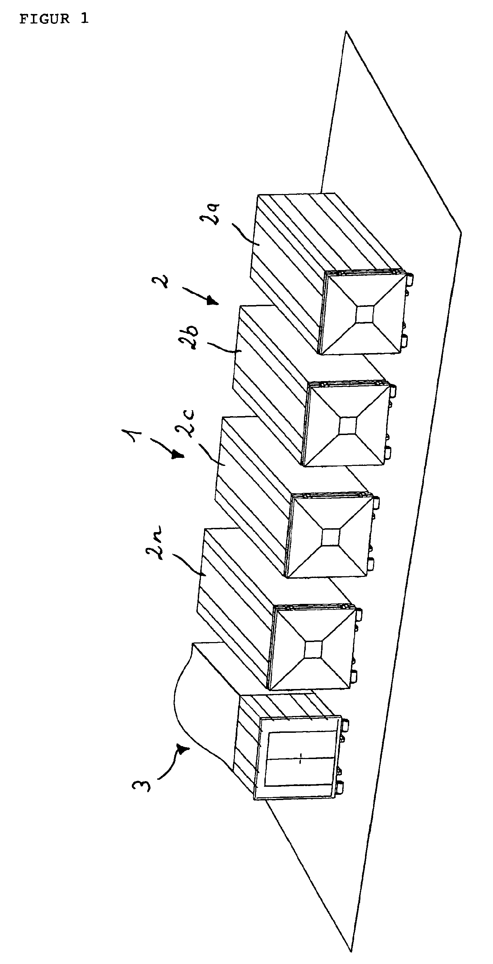

Wie Fig. 1 zeigt, besteht die Biogasanlage 1 vorteilhafterweise aus mehreren Modulen, deren Anzahl je nach Kapazitätsbedarf verändert werden kann. Die genannten Module sind dabei einerseits Reaktoreinheiten 2a, 2b, ..., 2n, sowie andererseits ein Technik- bzw. Steuerungsmodul, das nachfolgend als Steuereinheit 3 bezeichnet wird.

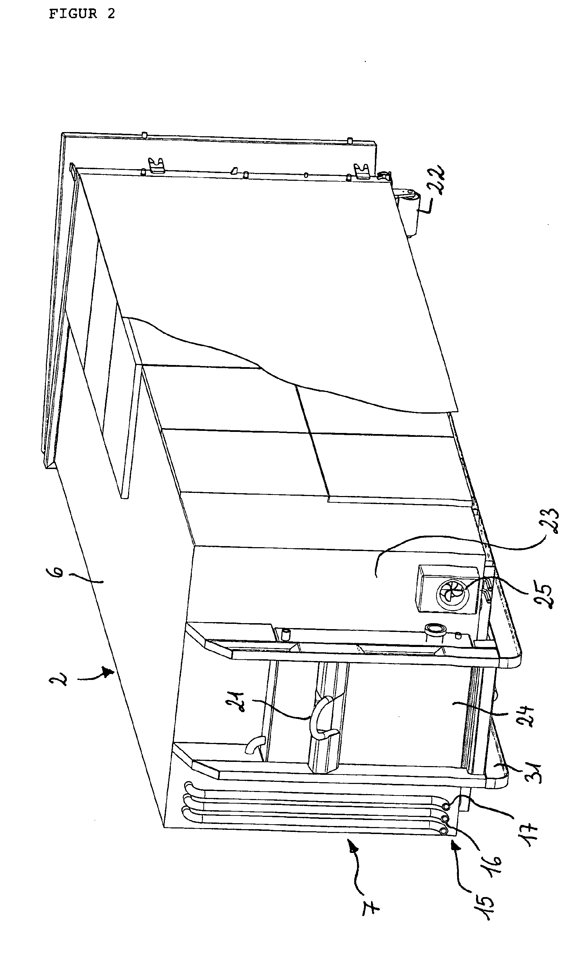

Die genannten Reaktoreinheiten 2 bilden jeweils Transportcontainereinheiten, die als Abroll-Hakencontainer ausgebildet sind, um von an sich bekannten Abroll-Hakencontainerlastfahrzeugen aufgeladen, transportiert und wieder abgeladen werden zu können. Wie Fig. 2 zeigt, umfassen die genannten Reaktoreinheiten 2 einen kubischen Containerkorpus, der als Reaktorbehälter 6 dient, in den zu verarbeitender Abfall gegeben und fermentiert werden kann. Der genannte Reaktorbehälter 6 ist hierbei mit einer geeigneten Befüllöffnung versehen, die beispielsweise in Form einer aufschwenkbaren Klappe oder Tür ausgebildet sein kann. Der Reaktorbehälter 6 sitzt in der gezeichneten Ausführungsform auf einem eine Stirnseite und den Boden umfassenden Tragrahmen 31, der bodenseitig zu den Stirnseiten hin mit Rollen 22 versehen ist, um am Boden abgerollt werden zu können. An der Stirnseite 23 des Reaktorbehälters 6 ist der Tragrahmen 31 mit einer Hakenaufnahme 21 versehen, um vom Haken der Hubvorrichtung des Lastfahrzeugs gegriffen werden zu können.

Der Reaktorbehälter 6 besitzt vorteilhafterweise die üblichen Standard-Containerabmessungen, um mit herkömmlichen Abrollcontainerfahrzeugen kompatibel und auf öffentlichen Straßen transportierbar zu sein.

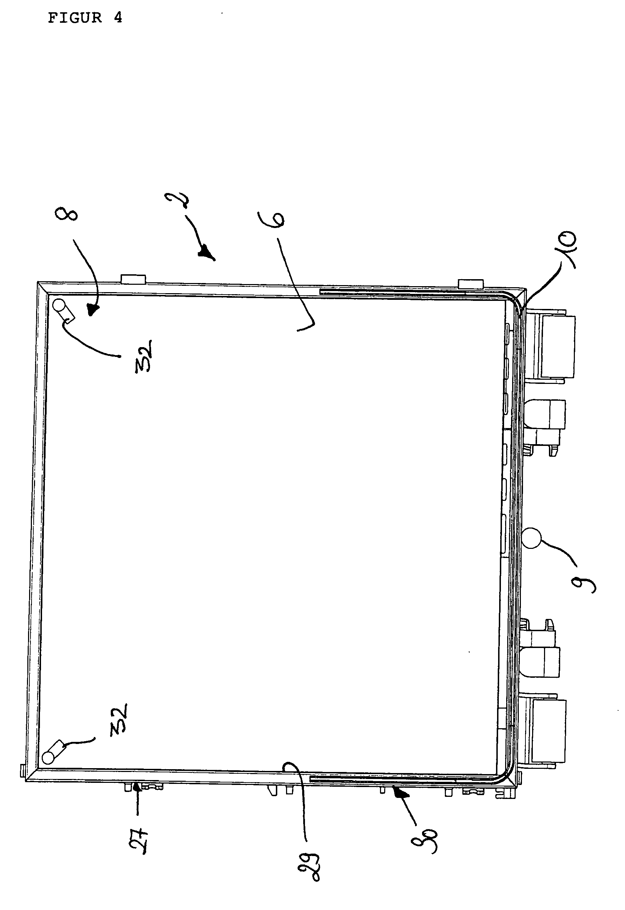

Die Reaktoreinheiten 2 umfassen neben dem genannten Reaktorbehälter 6 Funktionsaggregate 7, um den Vergasungs- bzw. Fermentierungsprozess des Abfalles auslösen und abwickeln zu können. In der gezeichneten Ausführung umfassen die genannten Funktionsaggregate 7 eine Perkolationseinrichtung 8, die es erlaubt, den Abfall im Inneren des Reaktorbehälters 6 mit Perkolat zu besprühen. Die genannte Perkolationsvorrichtung 8 umfasst hierzu einen Perkolationsmitteltank 24 auf der zuvor genannten Containerstirnseite 23, der mit im Innenraum des Reaktorbehälters 6 deckenseitig angebrachten Perkolationsleitungen 32 verbunden ist, vgl. Fig. 4. Über eine nicht eigens gezeigte Pumpe kann über die genannten Perkolationsleitungen 32 Perkolat eingesprüht werden.

Weiterhin umfassen die genannten Funktionsaggregate 7 eine Belüftungseinrichtung 9, die ein ebenfalls an der genannten Containerstirnseite 23 angebrachtes Gebläse 25 umfasst, um die Luftmenge im Reaktorbehälter 6 steuern zu können. Vorteilhafterweise kann die Belüftungseinrichtung 9 hierbei eine am Boden des Reaktorbehälters 6 angeordnete Belüftungsleitung 26 umfassen, über die von unten her Luft in den Reaktorbehälter 6 eingeblasen werden kann. Vorteilhafterweise kann die genannte Belüftungsleitung 26 gleichzeitig als Ablauf für bei dem Fermentierprozess entstehende Flüssigkeiten Verwendung finden.

Ferner umfassen die genannten Funktionsaggregate 7 eine nicht näher gezeigte Sensorik, mittels derer relevante Prozessparameter im Inneren des Reaktorbehälters 6 erfasst werden können. Insbesondere kann dies ein Gassensor sein, mittels dessen die Zusammensetzung des entstehenden Gases bestimmt werden kann. Alternativ oder zusätzlich kann ein Gasmengensensor vorgesehen sein, mittels dessen die Menge des entstehenden Gases erfasst werden kann.

Ferner ist an dem Boden des Reaktorbehälters 6 eine Heizeinrichtung 10 in Form einer Bodenheizung vorgesehen, um den Boden des Reaktorbehälters 6 und damit den im Innenraum befindlichen Abfall auf Temperatur bringen zu können.

Das bei dem Prozess entstehenden Gas kann aus dem Reaktorbehälter 6 über eine Gasleitung abgeleitet werden, die in der gezeichneten Ausführung ebenfalls an der Containerstirnseite 23 angeordnet ist und mit einer entsprechenden Gasanschlusskupplung 16 an den noch zu beschreibenden Gasspeicher angeschlossen werden kann.

Neben der genannten Gasanschlusskupplung 16 umfassen die Kupplungsmittel 15 der Reaktoreinheit 2 weiterhin eine Signalleitungs- und/oder Energieanschlusskupplung 17, über die die zuvor genannten Funktionsaggregate 7 mit der Steuereinheit 3 verbunden werden können. Die genannten Kupplungsmittel 15 sind vorteilhafterweise ebenfalls an der genannten Containerstirnseite 23 angeordnet, so dass ein zentraler Anschluss von nur einer Containerseite her möglich ist.

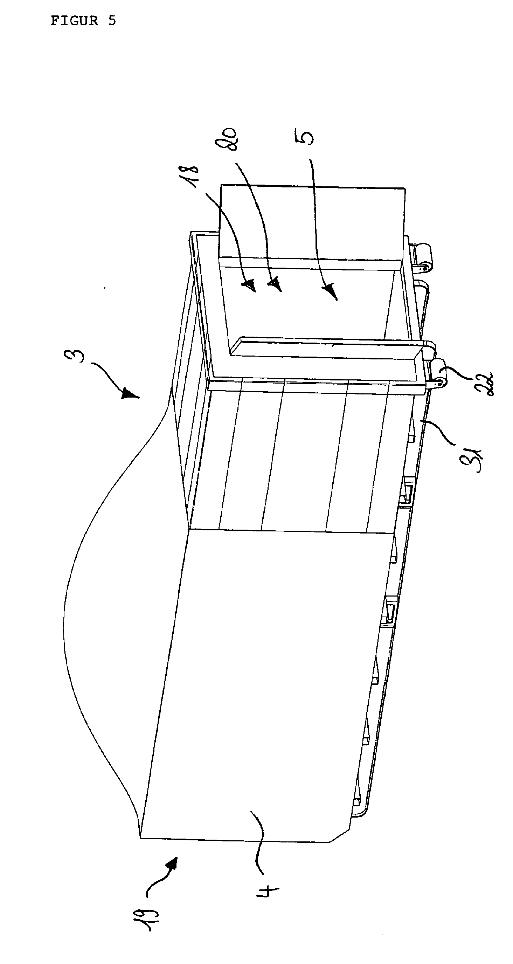

Die genannte Steuereinheit 3 ist vorteilhafterweise ebenfalls als Transportcontainereinheit ausgebildet und umfasst ein zentrales elektronisches Steuermodul 5 sowie einen Gasspeicher 4 zur Pufferung der aus den Reaktoren abgeleiteten Gasmenge. Die entsprechende in den Figuren 1 und 5 gezeigte Transportcontainereinheit umfasst ebenfalls einen kubischen Containerkorpus, der mit einem Tragrahmen 31 in der zuvor beschriebenen Weise versehen ist, um von einem Abrollhakencontainerfahrzeug aufgeladen, transportiert und wieder abgeladen werden zu können. Die Transportcontainereinheit ist hierbei in zwei Kammern unterteilt, von denen eine der Aufnahme des elektronischen Steuermoduls 5 und die andere der Aufnahme des Gasspeichers 4 dient. Wie Fig. 5 zeigt, kann der Gasspeicher 4 mit einer verformbaren Membran versehen sein, die die Decke des entsprechenden Containerabschnitts bildet.

Die Steuereinheit 3 ist mit einer Mehrzahl von Kupplungsmitteln 18 ausgerüstet, um eine entsprechende Mehrzahl von Reaktoreinheiten 2 anschließen zu können. Die genannten Kupplungsmittel 18 umfassen dabei jeweils eine Gasanschlusskupplung 19 sowie eine Signalleitungs- und Energieanschlusskupplung 20, um die entsprechenden Leitungen bzw. Kupplungen der Reaktoreinheiten anschließen zu können.

Die Reaktorbehälter 6 sind vorteilhafterweise mit mehrschichtig aufgebauten Wandungen versehen, die eine Wärmeisolationsschicht 27 besitzen, um weniger Energie für die Ingangsetzung des Prozesses verwenden zu müssen. Die genannte Wärmeisolationsschicht 27 kann beispielsweise in Form von Schaumstoffplatten ausgebildet sein, die auf einer innen liegenden Stützschicht 28 aufgebracht sind. Die genannte Stützschicht 28 kann hierbei von der Innenwandung 29 des Containerkorpus gebildet sein, die vorteilhafterweise aus Metallblech bestehen kann. Die Wärmeisolationsschicht 27 ist außenseitig vorteilhafterweise durch beschichtete Außenbleche verkleidet, die die Außenwandung 30 bilden. Hierdurch ist die stoßanfällige Wärmeisolationsschicht 27 geschützt.

Je nach Kapazitätsbedarf wird die erforderliche Anzahl an Reaktoreinheiten 2 an die Steuereinheit 3 angeschlossen, wobei der Prozess in den jeweiligen Reaktoreinheiten grundsätzlich in verschiedener Weise ablaufen bzw. gesteuert werden kann. Beispielsweise kann die Gasgewinnung in mehreren aufeinander folgenden Prozessphasen betrieben werden. Insbesondere kann in einer ersten Prozessphase unter aeroben Bedingungen gearbeitet werden. Das Substrat im Bioreaktor wird hierbei so lange belüftet, bis die vorgewählte Zieltemperatur durch mikrobielle Selbsterhitzung erreicht wird. Nach Erreichen der Zieltemperatur wird die Belüftung gestoppt und der restliche Sauerstoff innerhalb weniger Stunden von den aeroben Mikroorganismen verbraucht. Basis für die Regelung der Belüftungsintensität kann eine permanente Messung des Sauerstoffgehalts im Reaktor sein.

In einer zweiten Prozessphase kann unter anaeroben Verhältnissen gearbeitet werden, wobei diese Prozessphase durch Animpfen des Substrats mit Anaerobbakterien gestartet werden kann. Hierzu wird Perkolat in regelmäßigen Intervallen in den Bioreaktor eingesprüht. Perkolat ist Sickerwasser, das prozessbedingt entsteht und in beheizten Behältern gespeichert wird. Die Methanbildung beginnt hierbei üblicherweise innerhalb von wenigen Stunden nach Start der genannten zweiten Prozessphase und kann im mesophilen oder im thermophilen Temperaturbereich ablaufen. Im mesophilen Temperaturbereich kann eine Temperatur von etwa 38° herrschen, während im thermophilen Bereich eine Temperatur von etwa 55° herrscht. Die Methankonzentration im Bioreaktor kann kontinuierlich bis über 70 % ansteigen, wobei die Verwertung des Biogases in Gasbrennern oder Blockheizkraftwerken jedoch auch schon bei niedrigerer Methankonzentration beginnen kann, insbesondere dann, wenn es mit hochkonzentriertem Biogas aus parallel laufenden Bioreaktoren vermischt wird.

In der genannten zweiten Prozessphase wird die Gaszusammensetzung vorteilhafterweise ebenfalls kontinuierlich oder wiederholt gemessen, um einerseits den Prozess steuern zu können und andererseits die Lieferung von verwertbarem Gas sicherzustellen. Zusätzlich kann die Sensorik die Temperatur und den Druck in den Reaktoren überwachen. Zur Beendigung der genannten zweiten Prozessphase und damit der Methanproduktion kann die Perkolation beendet und/oder die Belüftung gestartet werden.

In einer dritten Prozessphase kann wieder unter rein aeroben Bedingungen gearbeitet werden. Sauerstoffgehalt, Druck und Temperatur im Reaktor werden vorteilhafterweise weiterhin gemessen, um sicherzustellen, dass der Prozess entsprechend den Vorgaben für die Zielqualität des Endprodukts abläuft. Ziele der dritten Prozessphase können z.B. die Hygienisierung des Materials durch Rottetemperaturen über 65°C oder die Entfeuchtung des Substrats bis zur Siebfähigkeit sein.

## Patentansprüche

1. Biogasanlage zur Erzeugung von Biogas aus organischen Abfällen, mit zumindest einer Reaktoreinheit (2), die einen Reaktorbehälter (6) zur Aufnahme des zu behandelnden Abfalls sowie Funktionsaggregate (7) wie eine Perkolationseinrichtung (8) zur Zugabe von Perkolat, eine Belüftungseinrichtung (9) zur Steuerung der Luftmenge im Reaktorbehälter (6), eine Heizeinrichtung (10) zur Beheizung des im Reaktorbehälter (5) befindlichen Abfalles und/oder eine Sensoreinrichtung (11) zur Erfassung zumindest eines im Reaktorbehälter herrschenden Prozessparameters aufweist, ferner mit einem Gasspeicher (4) zur Zwischenspeicherung des erzeugten Gases sowie einer Steuereinheit (3) zur Steuerung und/oder Überwachung der Funktionsaggregate (7) der Reaktoreinheit (2), **dadurch gekennzeichnet, dass** die Reaktoreinheit (2) als mobile Fahrzeugcontainereinheit ausgebildet ist, die mit lösbaren Kupplungsmitteln (15) umfassend eine Gasanschlusskupplung (16) zum Ankuppeln an den Gasspeicher (4) und eine Signalleitungs- und/oder Energieanschlusskupplung (17) zum Ankuppeln an die Steuereinheit (3) versehen ist.

2. Biogasanlage nach dem vorhergehenden Anspruch, wobei der Gasspeicher (4) und/oder die Steuereinheit (3) in Form einer Fahrzeugcontainereinheit ausgebildet sind, die mit lösbaren Kupplungsmitteln (18) umfassend eine Mehrzahl von Gasanschlusskupplungen (19) und/oder mehreren Signalleitungs- und/oder Energieanschlusskupplungen (20) zum Ankuppeln mehrerer Reaktoreinheiten (2) versehen ist.

3. Biogasanlage nach dem vorhergehenden Anspruch, wobei der Gasspeicher (4) und/oder die Steuereinheit (3) voneinander getrennt in Form separater Funktionsbausteine ausgebildet sind und verschiedene Abschnitte der Fahrzeugcontainereinheit bilden.

4. Biogasanlage nach einem der Ansprüche 2 oder 3, wobei der Gasspeicher (4) in einer Containerkammer untergebracht ist, die zumindest abschnittsweise durch eine verformbare Membran verschlossen ist.

5. Biogasanlage nach einem der vorhergehenden Ansprüche, wobei die Steuereinheit (3) einen Identifikationsbaustein zur automatischen Identifikation einer an die Steuereinheit (3) angeschlossenen Reaktoreinheit (2) und einen Anpassungsbaustein zur automatischen Anpassung der bereitgestellten Steuerbefehle in Abhängigkeit einer jeweils identifizierten Reaktoreinheit aufweist.

6. Biogasanlage nach einem der vorhergehenden Ansprüche, wobei an einem Boden der Fahrzeugcontainereinheit die Heizeinrichtung (10) zum Beheizen des zu behandelnden Abfalls und eine Belüftungsleitung (26) zur Belüftung des Containerinnenraums vorgesehen sind.

7. Biogasanlage nach einem der vorhergehenden Ansprüche, wobei zumindest ein Teil der Funktionsaggregate (7) und/oder ein Teil der Kupplungsmittel (15; 18) an einer Containerstirnseite (23) angeordnet sind, an der eine Containerhubkupplung zum Anheben der Fahrzeugcontainereinheit vorgesehen ist.

8. Biogasanlage nach dem vorhergehenden Anspruch, wobei ein Perkolationsmitteltank (24) und Gasanschlüsse sowie ggf. auch ein Ventilator (25) an der genannten Containerstirnseite (23) der Fahrzeugcontainereinheit angeordnet sind.

9. Biogasanlage nach einem der vorhergehenden Ansprüche, wobei die Fahrzeugcontainereinheit zumindest abschnittsweise eine mehrschichtige Wandung umfassend eine Wärmeisolationsschnitt (27), vorzugsweise aus einem Schaumstoff, und eine Stabilität gebende Stützschicht (28), vorzugsweise aus einem druck- und/oder schlagfesten Material, aufweist, vorzugsweise mit einem sandwichartigen Aufbau umfassend eine Innenwandung (29) aus Metallblech, eine Außenwandung sowie eine Zwischen den genannten Innen- und Außenwandungen angeordneten Isolationsschicht (27).

10. Verfahren zum Betreiben einer Biogasanlage zur Erzeugung von Biogas aus organischen Abfällen, die eine Mehrzahl von Reaktoreinheiten (2) sowie eine Steuereinheit (3) zur Steuerung der Reaktoreinheiten (2) umfasst, **dadurch gekennzeichnet, dass** in Abhängigkeit des Kapazitätsbedarfs und/oder der anfallenden Abfallmenge die Anzahl der Reaktoreinheiten variiert wird, wobei bei steigendem Kapazitätsbedarf und/oder bei steigender Abfallmenge zumindest eine zusätzliche mobile Reaktoreinheit (2) angeliefert, mit der Steuereinheit (3) verbunden und in Betrieb genommen wird und bei fallendem Kapazitätsbedarf und/oder fallender Abfallmenge zumindest eine Reaktoreinheit (2) von der Steuereinheit (3) getrennt und abtransportiert wird.

11. Reaktoreinheit einer Biogasanlage zur Erzeugung von Biogas aus organischen Abfällen, mit einem Reaktorbehälter (6) zur Aufnahme des zu behandelnden Abfalls sowie Funktionsaggregaten (7) zur Behandlung und/oder Überwachung des im Reaktorbehälter (6) aufgenommenen Abfalls und dessen Prozessparameters, **dadurch gekennzeichnet, dass** die Reaktoreinheit als mobile Fahrzeugcontainereinheit ausgebildet ist, die mit lösbaren Kupplungsmitteln (15) umfassend eine Gasanschlusskupplung (16) zum Ankuppeln an einen Gasspeicher und eine Signalleitungs- und/oder Energieversorgungskupplung (17) zum Ankuppeln an eine Steuereinheit (3) versehen ist.

12. Reaktoreinheit nach dem vorhergehenden Anspruch, wobei die Fahrzeugcontainereinheit als Hakencontainer, vorzugsweise Abroll-Hakencontainer mit Hakenaufnahme (21) und Rollen (22), ausgebildet und/oder kompatibel zu einem Abroll-Containertransportlastfahrzeug ausgebildet ist.
